# FASCICULE DE BREVET EUROPEEN

(11) **EP 4 593 754 B1**
(45) Date de publication et mention de la délivrance du brevet: **16.09.2026**
(21) Numéro de dépôt: 23800501.1
(22) Date de dépôt: 28.09.2023
(51) Int. Cl.: A61B 50/30, A61B 50/33, A61B 1/00, A61B 17/00, A61B 50/00

(54) **EMBALLAGE POUR UN ENDOSCOPE MÉDICAL**
VERPACKUNG FÜR EIN MEDIZINISCHES ENDOSKOP
PACKAGING FOR A MEDICAL ENDOSCOPE

(30) Priorité: 29.09.2022 FR 2209870
(43) Date de publication de la demande: 06.08.2025
(73) Titulaire: Axess Vision Technology, 37300 Joue les Tours (FR)
(72) Inventeur: HALLAUER, Emmanuel, 37190 SACHE (FR); LEROUX, Philippe, 37100 TOURS (FR); LECOUTEY, Luka, 37210 ROCHECORBON (FR); COURATIN, Jérôme, 37700 SAINT-PIERRE-DES-CORPS (FR)
(74) Mandataire: Hindles Limited
(86) Numéro de dépôt international: PCT/FR2023/051492
(87) Numéro de publication internationale: WO 2024/069102

(56) Documents cités:
- EP-A1- 0 647 425
- EP-A2- 2 335 636
- GB-A- 2 497 353
- US-A- 4 262 800
- US-A- 5 947 284
- US-A1- 2020 214 820

## Description

### Domaine Technique

L'invention concerne un emballage d'un endoscope médical, à caractère réutilisable ou à usage unique.

Les endoscopes médicaux permettent d'accéder à la surface interne d'un organe creux, d'une cavité ou d'un conduit naturel ou artificiel du corps humain en vue d'effectuer diverses opérations à des fins thérapeutiques, chirurgicales ou de diagnostic. Les endoscopes médicaux sont utilisés pour l'inspection de toutes les parties internes du corps humain accessibles par les voies naturelles ou artificielles. Par exemple, les endoscopes médicaux peuvent être utilisés dans le domaine des voies urinaires, des voies gastro-intestinales, du système respiratoire, du système cardiovasculaire, de la trachée, de la cavité du sinus, du système de reproduction de la femme, de la cavité abdominale ou de tout autre partie du corps humain à explorer par une voie naturelle ou artificielle.

Au vu de leur utilisation, il est indispensable que les endoscopes médicaux soient stockés dans des emballages stériles, qui constituent une barrière stérile et qui les protègent d'éventuelles détériorations pouvant résulter de chocs lors de leur transport notamment.

### Technique antérieure

Un endoscope médical comprend une poignée de commande, un tube d'insertion et un câble d'alimentation. De manière usuelle, il est emballé dans une barquette ou plateau recouvert d'un film plastique. Typiquement, le plateau comprend un logement de réception de l'endoscope médical dont une partie permet de recevoir la poignée de commande qui se trouve dans le prolongement d'une seconde partie permettant de recevoir le tube d'insertion et éventuellement d'une troisième partie permettant de recevoir le câble d'alimentation. Ce plateau est recouvert d'un film plastique permettant la stérilisation et constituant une barrière stérile. En général, le film est fixé hermétiquement sur le pourtour du plateau pour assurer la stérilité de l'endoscope médical.

De manière classique, le logement comprend une nervure en forme d'arc de cercle ou circulaire pour recevoir le tube d'insertion, tel que cela ressort des brevets US 5,947,284 ou EP 3 148 396 notamment. Cependant, le tube d'insertion peut se déformer et s'endommager lorsqu'il est stocké dans cette position, et il est préférable de disposer d'un emballage dans lequel le tube est maintenu dans une position rectiligne dans le prolongement de la poignée de commande.

Afin de maintenir en position l'endoscope médical dans l'emballage, celui-ci peut comprendre des clips par exemple. Cependant, l'insertion ou le retrait de l'endoscope médical de son logement peut être difficile, et lorsque les clips se trouvent au niveau du tube d'insertion, ce dernier peut être endommagé lors de ces opérations d'insertion et de retrait. Afin de résoudre ce problème, diverses solutions ont été envisagées. Par exemple, le brevet US 5,947,284 propose d'insérer le tube d'insertion de l'endoscope médical dans un tube en forme d'arc de cercle qui sert alors de guide pour le retrait du tube d'insertion. Cependant, cette solution présente l'inconvénient de créer beaucoup de vibrations et de contraintes, en particulier pendant le transport. De plus, cette solution peut être difficile de mise en œuvre pour le praticien, non seulement pour sortir l'endoscope médical de son emballage, mais aussi s'il souhaite le poser lors de son utilisation. De plus, cette solution n'est pas économique puisqu'elle nécessite la préparation d'un tube adapté à l'emballage. Enfin, le tube d'insertion est stocké dans une position en forme d'arc de cercle, ce qui présente les risques mentionnés ci-dessus.

Ce même brevet US 5,947,284 propose une autre solution pour maintenir en position l'endoscope médical dans l'emballage, à savoir l'emploi d'un second film pour couvrir la partie du logement contenant le tube d'insertion. Pour cela, des points de fixation de ce second film sont disposés sur le pourtour du plateau, à proximité de la plage périphérique. Cette solution présente l'inconvénient de ne pas maintenir suffisamment bien en position le tube d'insertion dans l'emballage. De plus, cette solution peut être difficile de mise en œuvre pour le praticien en raison des deux films à retirer pour sortir l'endoscope de son emballage. Là encore, le tube d'insertion est placé dans un logement en forme d'arc de cercle, ce qui présente les inconvénients mentionnés ci-dessus. En outre, le document EP 0 647 425 divulgue une gaine endoscopique destinée à protéger et/ou à modifier un angle de vue d'un endoscope. La gaine comporte une partie distale conçue pour s'adapter à la partie la plus distale des optiques d'imagerie de l'endoscope. Cette partie distale intègre une structure permettant de modifier l'angle de vue de l'endoscope. Le document US 4,262,800 divulgue un support stérilisable pour une unité chirurgicale à fibre optique ou un instrument médical délicat similaire. Ce support comprend une base, un contour de l'instrument formé sur la base, des paires de tenons de retenue disposés autour du contour sur la base, ainsi que des bandes élastiques fixées aux tenons pour maintenir l'instrument sur le support. Le document GB 2,497,353 divulgue un dispositif de stockage conçu pour supporter un endoscope comportant un premier tube flexible destiné à être inséré dans le corps, un deuxième tube de transmission flexible, et un boîtier d'interface reliant les premier et deuxième tubes flexibles. Le plateau de stockage est conçu pour maintenir l'endoscope dans une configuration enroulée, de sorte qu'au moins l'un des deux tubes chevauche le boîtier d'interface. Le document US 2020/214820 divulgue un système d'emballage destiné à être utilisé avec un stent urétéral comportant un corps allongé s'étendant entre une extrémité proximale et une extrémité distale, et pouvant comprendre une base de plateau. La base de plateau peut comprendre au moins un premier tenon, un deuxième tenon, ainsi qu'une saillie ou une rainure conçue pour fixer respectivement l'extrémité distale, l'extrémité proximale et un lien du stent urétéral sur la base du plateau. Le document EP 2 335 636 divulgue un emballage stérilisable comprenant un élément de support et un matériau en feuille formant soit un couvercle pour l'élément de support, soit une pochette dans laquelle l'élément de support est disposé. Le matériau en feuille comprend un matériau barrière et inclut une membrane respirante constituée d'un matériau perméable à l'humidité et aux gaz, mais imperméable aux micro-organismes. La membrane respirante permet l'introduction d'un gaz de stérilisation dans l'emballage stérilisable. Elle est disposée vers une portion centrale du matériau en feuille et est espacée de toute soudure reliant le matériau en feuille à l'élément de support ou des soudures définissant la pochette.

Il existe donc un besoin de disposer d'un emballage pour endoscope médical qui soit stérilisable et qui soit suffisamment robuste pour constituer une barrière stérile jusqu'à son utilisation. Il existe également un besoin d'un emballage facile d'utilisation : son ouverture est avantageusement aisée, et permet de préférence au praticien de déposer facilement l'endoscope médical pendant son utilisation pour pouvoir le réutiliser ensuite. Il existe également un besoin pour un emballage permettant de maintenir en position de manière sécurisée l'endoscope médical : il est préférable que la poignée de commande et le tube d'insertion soient correctement maintenus en position dans leur logement afin qu'ils ne soient pas endommagés pendant leur stockage ou leur transport. Il existe également un besoin d'un emballage permettant de conserver le tube d'insertion en position rectiligne, et dans le prolongement de la poignée de commande.

### Exposé de l'invention

L'invention vise à remédier aux inconvénients de l'art de la technique en proposant un emballage d'un endoscope médical comportant une poignée de commande et un tube d'insertion , ledit emballage comprenant :
- un plateau pourvu d'une face interne présentant une plage périphérique, ledit plateau comportant sur sa face interne un logement recevant l'endoscope médical, le logement comprenant une cavité recevant la poignée de commande, et une gorge rectiligne recevant le tube d'insertion, la gorge présentant une section supérieure à la section du tube d'insertion pour permettre sa libre insertion,
- une feuille perméable aux gaz et aux rayonnements béta et gamma, imperméable aux microbes, scellée continument et hermétiquement à la plage périphérique de la face interne du plateau selon un effort d'arrachement, caractérisé en ce que le plateau comprend des protubérances s'étendant en saillie à partir de sa face interne et disposées de part et d'autre de la gorge, et en ce que la feuille est fixée aux protubérances selon un effort d'arrachement inférieur d'au moins 30% par rapport à l'effort d'arrachement entre la feuille et la plage périphérique .

Dans le cadre de l'invention, l'effort d'arrachement entre la feuille et la plage périphérique (ou les protubérances) correspond à la force nécessaire par unité de surface pour arracher la feuille de la plage périphérique (ou des protubérances). L'effort d'arrachement peut être mesuré en N/mm².

Dans le cadre de l'invention, un « effort d'arrachement entre la feuille et les protubérances inférieur d'au moins 30% par rapport à l'effort d'arrachement entre la feuille et la plage périphérique » signifie que le ratio entre l'effort d'arrachement entre la feuille et les protubérances et l'effort d'arrachement entre la feuille et la plage périphérique à isosurface (c'est-à-dire pour une même surface de contact entre la feuille et les protubérances d'une part, et entre la feuille et la plage périphérique d'autre part) est inférieur ou égal à 0,7.

L'emballage selon l'invention permet de maintenir en position le tube d'insertion rectiligne et dans le prolongement de la poignée de commande, et ainsi assurer une meilleure préservation du tube d'insertion.

L'emballage selon l'invention permet d'assurer une barrière stérile pour l'endoscope médical, grâce au scellage continu et hermétique entre le plateau et la feuille qui est imperméable aux microbes.

La présence de protubérances de part et d'autre de la gorge permet de maintenir en position le tube d'insertion dans l'emballage, sans déformation ou écrasement.

L'emballage selon l'invention est facile à utiliser, tant pour la mise en place de l'endoscope dans son emballage, que pour l'ouverture de l'emballage par le praticien. L'ouverture est aisée et rapide. Selon un mode de réalisation avantageux de l'invention, le praticien peut reposer l'endoscope médical dans son emballage au cours de son intervention si besoin, et l'utiliser de nouveau ensuite pour le même patient et au cours de la même intervention par exemple.

L'emballage selon l'invention présente avantageusement l'une ou l'autre des caractéristiques suivantes, ou une combinaison de celles-ci :
- les protubérances sont positionnées en bordure de la gorge ;
- les protubérances sont réparties le long de la gorge ;
- les protubérances sont disposées alternativement d'un côté ou de l'autre de la gorge ;
- le logement comprend une gouttière recevant un câble d'alimentation de l'endoscope médical ;
- au moins une protubérance est positionnée en bordure de la gouttière ;
- au moins une protubérance est présente dans la gouttière afin d'enrouler le câble d'alimentation ;
- les protubérances sont des plots ou des nervures ;
- l'emballage comprend deux lignes d'arrêt aménagées dans les parties de la plage périphérique situées de part et d'autre de la gorge ;
- le logement comporte des clips pour maintenir en position la poignée de commande ;
- le logement comprend une alvéole recevant un connecteur du câble d'alimentation de l'endoscope médical ;
- l'emballage comprend une fente d'accrochage dans la plage périphérique ;
- l'emballage comprend en outre une surface adhésive sur la face interne du plateau qui est recouverte d'un film pelable.

L'invention concerne également un procédé d'emballage d'un endoscope médical comprenant les étapes suivantes :
a) disposer d'un endoscope médical comportant une poignée de commande, un tube d'insertion, et éventuellement un câble d'alimentation,
b) disposer d'un plateau pourvu d'une face interne présentant une plage périphérique, ledit plateau comportant sur sa face interne un logement destiné à recevoir un endoscope médical, le logement étant composé d'une cavité destinée à recevoir la poignée de commande, d'une gorge rectiligne destinée à recevoir le tube d'insertion, et éventuellement d'une gouttière destinée à recevoir le câble d'alimentation, la gorge présentant une section supérieure à la section du tube d'insertion pour permettre sa libre insertion, ledit plateau comprenant des protubérances s'étendant en saillie à partir de sa face interne et disposées de part et d'autre de la gorge,
c) disposer d'une feuille perméable aux gaz et aux rayonnements béta et gamma, imperméable aux microbes et thermoscellable,
d) placer l'endoscope médical dans le logement,
e) mettre en contact la feuille thermoscellable avec la plage périphérique et avec les protubérances, chauffer à une température de scellage, appliquer une pression de scellage périphérique sur la plage périphérique permettant un scellage hermétique de la feuille sur la plage périphérique pour obtenir un effort d'arrachement, et appliquer une pression de scellage central sur les protubérances permettant une fixation de la feuille sur les protubérances pour obtenir un effort d'arrachement, la pression de scellage périphérique et la pression de scellage central étant telles que l'effort d'arrachement entre la feuille et les protubérances est inférieur d'au moins 30% par rapport à l'effort d'arrachement entre la feuille et la plage périphérique.

Avantageusement, l'étape b) du procédé selon l'invention est telle que soit la plage périphérique et les protubérances s'étendent en saillie selon une même hauteur par rapport à la surface interne du plateau, soit les protubérances sont en saillie selon une hauteur inférieure à celle de la plage périphérique par rapport à la surface interne du plateau.

### Brève description des dessins

[Fig. 1] La figure 1 est une vue du dessus montrant un plateau d'un emballage pour recevoir un endoscope conformément à une première variante de réalisation de l'invention selon laquelle les protubérances sont sous forme de plots.
[Fig. 2] La figure 2 est une vue de dessus montrant un endoscope médical dans un emballage conformément à une première variante de réalisation de l'invention selon laquelle les protubérances sont sous forme de plots.
[Fig. 3] La figure 3 est une vue en coupe selon la ligne BB de la figure 2 montrant le tube d'insertion d'un endoscope médical dans la gorge d'un emballage conforme à l'invention.
[Fig. 4] La figure 4 représente une vue de détail de vue de dessus montrant un emballage conforme à une deuxième variante de réalisation de l'invention selon lequel les protubérances sont sous forme de nervures et de plots.
[Fig. 5] La figure 5 est une vue du dessus montrant un endoscope médical dans un emballage conformément à la deuxième variante de réalisation de l'invention dans laquelle les protubérances sont sous forme de plots et de nervures.
[Fig. 6] La figure 6 est une vue en coupe prise selon les lignes AA de la figure 5.
[Fig. 7] La figure 7 est une vue en coupe prise selon les lignes II de la figure 5.
[Fig. 8] La figure 8 est une vue de dessus montrant un endoscope médical dans un emballage, comprenant en outre une alvéole pour le connecteur du câble.
[Fig. 9] La figure 9 est une vue de dessus montrant un endoscope médical dans un emballage, comprenant une surface adhésive permettant de refermer l'emballage après son ouverture.

### Description des modes de réalisation

Tel que représenté dans les figures, l'invention concerne un emballage 1 pour un endoscope médical 2, composé de manière classique d'une poignée de commande 3, d'un tube d'insertion 4 et éventuellement d'un câble d'alimentation 5. L'emballage 1 selon l'invention comporte un plateau 6 aménagé pour recevoir l'endoscope médical 2 et fermé par une feuille 7 assemblée avec le plateau 6.

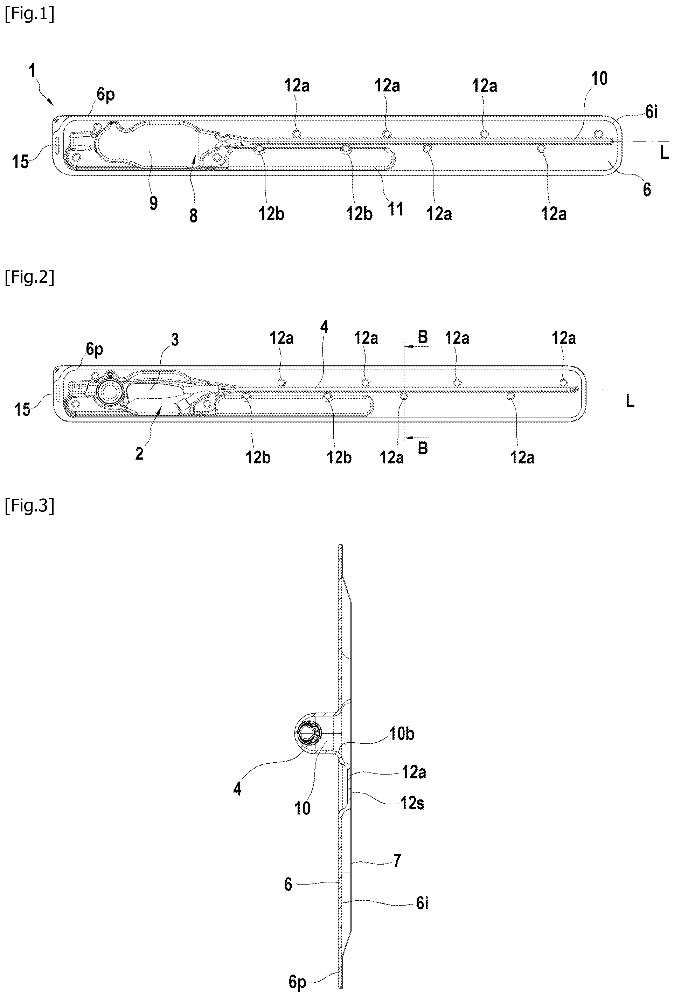

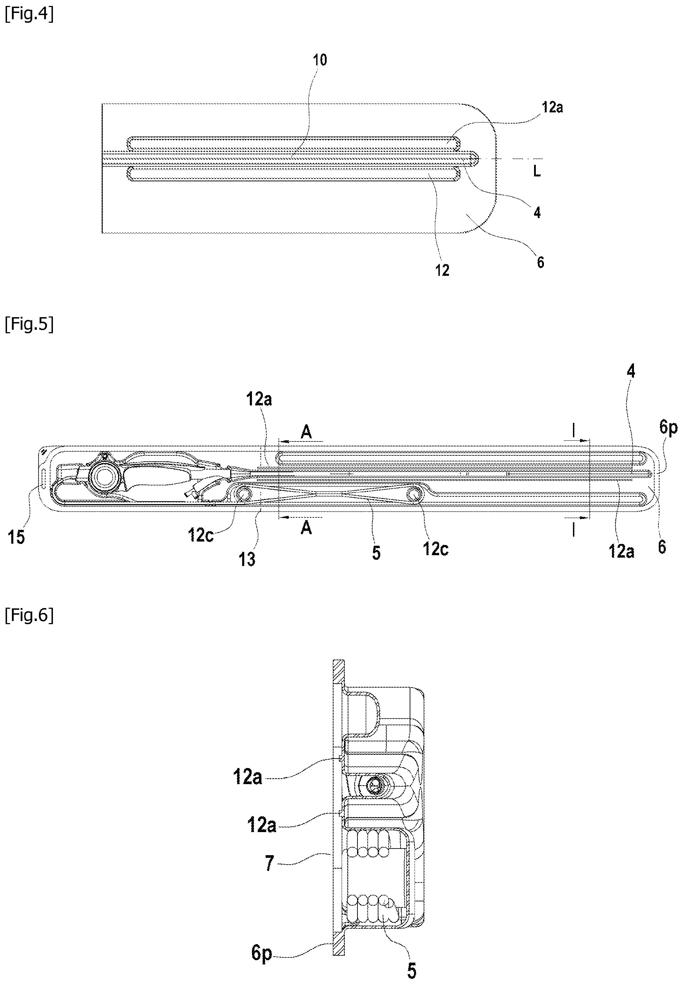

Tel que représenté à la figure 1, le plateau 6 est pourvu d'une face interne 6ᵢ présentant une plage périphérique 6ₚ. Selon un premier mode de réalisation illustré aux figures 2 et 3, la plage périphérique 6ₚ ne dépasse pas de la face interne 6ᵢ du plateau 6 mais est à fleur de la face interne 6ᵢ, c'est-à-dire que la plage périphérique 6ₚ et la face interne 6ᵢ du plateau 6 appartiennent à un même plan. Alternativement, selon le mode de réalisation illustré aux figures 5 à 7, la plage périphérique 6ₚ s'étend en saillie à partir de la face interne 6ᵢ du plateau 6, formant ainsi un rebord périphérique.

Le plateau 6 comporte un logement 8 sur sa face interne 6ᵢ afin de recevoir l'endoscope médical 2, tel qu'illustré à la figure 1. Ce logement 8 se compose d'une cavité 9 débouchant dans une gorge rectiligne 10, ainsi que dans une gouttière 11. Bien que non illustré, le logement 8 peut ne pas comporter de gouttière 11 pour le cas où l'endoscope médical 2 ne comporte pas un câble d'alimentation 5.

La cavité 9 est destinée à recevoir la poignée de commande 3. Ses dimensions permettent d'insérer totalement la poignée de commande 3 de sorte que celle-ci ne dépasse pas de la face interne 6ᵢ du plateau 6. Selon un mode de réalisation particulier de l'invention, la cavité 9 comprend au moins un clip pour maintenir en position la poignée de commande 3.

La gouttière 11 est destinée à recevoir le câble d'alimention 5. Les dimensions et la forme de la gouttière 11 sont telles que la totalité du câble d'alimentation 5 peut y être insérée, de sorte que celui-ci ne dépasse pas de la surface interne 6ᵢ du plateau 6.

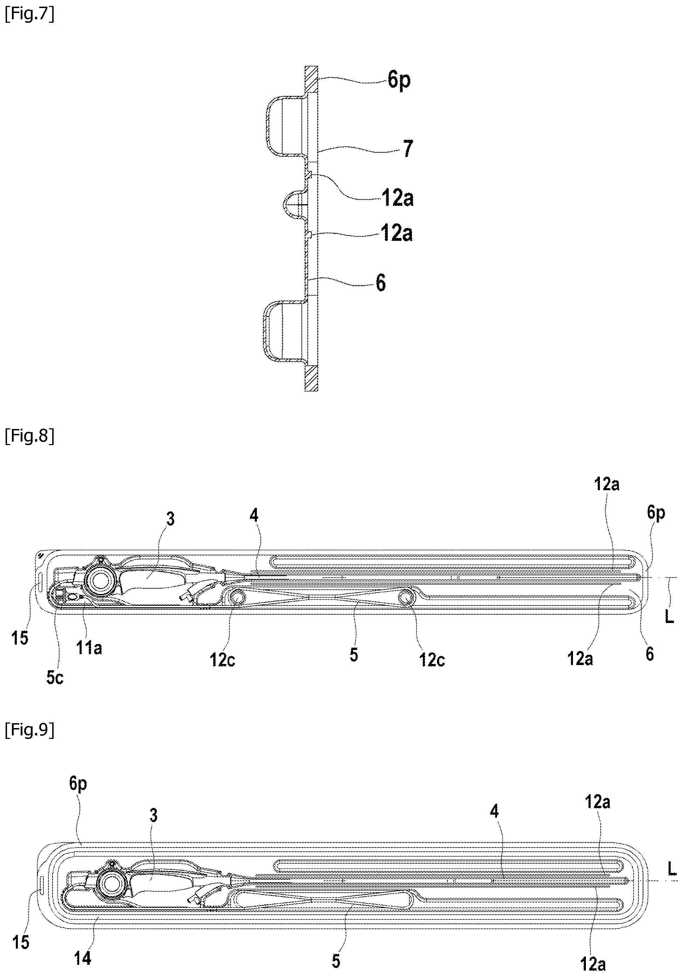

Selon un mode de réalisation illustré à la figure 8, le câble d'alimentation 5 est pourvu à son extrémité libre un connecteur 5_{c}. Selon ce mode de réalisation, le logement 8 comprend en outre une alvéole 11ₐ destinée à recevoir le connecteur 5_{c}. Plus précisément, la gouttière 11 débouche dans l'alvéole 11ₐ. L'alvéole 11ₐ peut comporter au moins un clip pour maintenir en position le connecteur 5_{c}. Selon ce mode de réalisation, le connecteur 5_{c} ne dépasse pas de la surface interne 6ᵢ du plateau 6.

La gorge 10 est destinée à recevoir le tube d'insertion 4 de l'endoscope médical 2. La gorge 10 présente donc une section supérieure à la section du tube d'insertion 2 pour permettre sa libre insertion. Avantageusement, la section de la gorge 10 est supérieure à la section du tube d'insertion avec un jeu d'insertion permettant un libre engagement sans coincement du tube d'insertion 4 dans la gorge 10. Ainsi le tube d'insertion 4 est maintenu latéralement par les parois de la gorge 10 tout en pouvant être inséré ou retiré sans blocage. Les risques d'endommagement du tube d'insertion 4 lors des opérations de montage et de retrait sont ainsi évités. La gorge 10 est rectiligne selon un axe longitudinal L, de sorte que le tube d'insertion 4 est également rectiligne lorsqu'il est placé dans l'emballage 1. De préférence, la gorge 10 est de forme tubulaire. La gorge 10 s'ouvre sur la face interne 6ᵢ par son bord 10_{b}, tel qu'illustré à la figure 3.

Le plateau 6 comprend également au moins deux protubérances présentant une face supérieure 12ₛ et s'étendant en saillie à partir de la face interne 6ᵢ du plateau 6.

Dans le cadre de l'invention, le plateau 6 comprend au moins deux protubérances 12ₐ disposées de part et d'autres de la gorge 10, c'est-à-dire de chaque côté de la gorge 10. En d'autres termes, ces protubérances 12ₐ s'étendent en saillie à partir de la face interne 6ᵢ du plateau 6, entre la plage périphérique 6ₚ et le logement 8. De préférence, ces protubérances 12ₐ sont positionnées en bordure de la gorge 10, c'est-à-dire que les protubérances 12ₐ sont jointives au bord 10_{b} de la gorge. Avantageusement, les protubérances 12ₐ sont contiguës à la gorge 10, et en particulier la distance entre chaque protubérance 12ₐ et le bord 10_{b} de la gorge 10 est inférieure au diamètre du tube d'insertion 4, ce qui permet d'éviter que le tube d'insertion 4 sorte de la gorge 10. Ces protubérances 12ₐ sont avantageusement réparties d'une extrémité à l'autre de la gorge 10, bien que cela ne soit pas obligatoire et qu'elles pourraient être disposées le long d'une partie de la gorge 10. De préférence, les protubérances 12ₐ sont disposées alternativement d'un côté ou de l'autre de la gorge 10. Bien que non préféré, il pourrait être envisagé que les protubérances 12ₐ soient disposées en vis-à-vis de part et d'autre de la gorge 10. De préférence, les protubérances 12ₐ sont réparties à intervalle régulier le long de la gorge 10. Selon un mode de réalisation, les protubérances 12ₐ sont alignées selon deux axes parallèles à l'axe longitudinal L de la gorge 10, ces deux axes étant à distance égale ou non de l'axe longitudinal L de la gorge 10.

Selon un mode de réalisation particulier de l'invention illustré en figure 2 notamment, le plateau 6 comprend en outre au moins une protubérance 12_{b} positionnée en bordure de la gouttière 11 afin de maintenir en position le câble d'alimentation 5 dans celle-ci. Ces protubérances 12_{b} peuvent venir situées de part et d'autre de la gouttière 11. Alternativement, tel que cela est représenté aux figures 1 et 2 notamment, la gouttière 11 est aménagée pour traverser un moins une protubérance 12_{b} de manière que l'au moins une protubérance 12_{b} s'étende de part et d'autre de la gouttière 11. Ainsi, une même protubérance 12_{b} peut être positionnée pour être en bordure à la fois la gorge 10 et de la gouttière 11.

Selon un mode de réalisation particulier de l'invention illustré en figure 5 notamment, le plateau 6 comprend en outre une ou plusieurs protubérances 12_{c} positionnées dans la gouttière 11 afin de maintenir en position le câble d'alimentation 5 dans la gouttière 11. Ainsi selon ce mode de réalisation, le câble d'alimentation 5 est enroulé autour des protubérances 12_{c} qui sont montées dans la gouttière 11.

Selon un premier mode de réalisation, les protubérances 12ₐ, 12_{b}, 12_{c} peuvent être des éléments discrets, et se présentent alors sous la forme de plots ou colonnettes, comme cela est représenté aux figures 1 à 3 notamment. Avantageusement, les différents plots bordant la gorge 10 et/ou la gouttière 11 sont de dimensions identiques, bien qu'une légère variation soit envisageable. Selon ce mode de réalisation, les protubérances 12_{c} montées dans la gouttière 11 peuvent avantageusement être de dimensions différentes des protubérances 12ₐ bordant la gorge 10 et des protubérances 12_{b} bordant la gouttière 11, bien que cela ne soit pas obligatoire.

Selon un second mode de réalisation, les protubérances 12ₐ, 12_{b}, 12_{c} se présentent sous la forme de nervures, de préférence rectilignes et parallèles à l'axe longitudinal L de la gorge 10, tel que cela est représenté aux figures 4 et 5. Les dimensions des nervures peuvent être différentes ou, de préférence, égales. Selon ce mode de réalisation, la gorge 10 et/ou la gouttière 11 peuvent être bordées par deux nervures allant de l'une de ses extrémités à l'autre, c'est-à-dire que chacune des deux nervures a une longueur identique au bord 10b de la gorge 10 et/ou au bord de la gouttière 11. Alternativement, la gorge 10 et/ou la gouttière 11 peut être bordée par au moins deux nervures de longueur inférieure à la longueur de la gorge 10 et/ou de la gouttière 11.

Selon un troisième mode de réalisation, certaines protubérances 12ₐ, 12_{b}, 12_{c} sont sous forme de plots, et certaines protubérances 12ₐ, 12_{b}, 12_{c} sont sous forme de nervures parallèles à l'axe longitudinal L. Ce mode de réalisation est notamment illustré aux figures 5 et 8.

Le plateau 6 peut être réalisé en tout matériau permettant non seulement sa fixation avec la feuille 7, mais permettant aussi de contribuer à la stérilité de l'endoscope médical 2. Le plateau 6 est typiquement réalisé en matériau thermoplastique, et par exemple choisi parmi le polyéthylène téréphtalate (PET), le polyéthylène téréphtalate amorphe (APET), le polyéthylène téréphtalate glycolisé (PETg), le polystyrène, et le polychlorure de vinyle (PVC).

L'emballage 1 selon l'invention comprend en outre une feuille ou film 7 fixée à la plage périphérique 6ₚ du plateau 6 et sur les protubérances 12ₐ, 12_{b}, 12_{c}. Cette fixation peut être réalisée par thermoscellage par exemple.

La plage périphérique 6ₚ du plateau 6 et la feuille 7 sont scellés. Ainsi, la fixation entre le plateau 6 et la feuille 7 est continue et hermétique sur tout le pourtour de la face interne 6ᵢ du plateau 6. Selon le mode de réalisation illustré à la figure 3, la plage périphérique 6ₚ ne dépasse pas de la face interne 6ᵢ du plateau 6 et la feuille 7 est déformée en direction du plateau. Selon le mode de réalisation illustré aux figures 6 et 7, la plage périphérique 6ₚ est saillante par rapport à la face interne 6ᵢ du plateau 6 et la feuille 7 est plane.

La fixation entre la feuille 7 et la plage périphérique 6ₚ du plateau 6 est définie par un effort d'arrachement pour arracher la feuille 7 de la plage périphérique 6ₚ du plateau 6. Cet effort d'arrachement est tel que la feuille peut être ôtée du plateau sans effort excessif par le praticien, tout en permettant une fermeture effective et stérile de l'emballage 1. Typiquement, l'effort d'arrachement entre la feuille 7 et la plage périphérique 6ₚ du plateau 6 va de à 0,0015 N/mm² à 2 N/mm², de préférence de 0,007 N/mm² à 0,03 N/mm². Dans le cadre de l'invention, l'effort d'arrachement peut être mesuré selon la norme EN 868-5.

Ensuite, la feuille 7 est également fixée au plateau 6 via les protubérances 12ₐ, 12_{b}, 12_{c}. Plus précisément, la feuille 7 est fixée sur la face supérieure 12ₛ des protubérances 12ₐ, 12_{b}, 12_{c}, quelle que soit leur forme. La fixation entre la feuille 7 et les protubérances 12ₐ, 12_{b}, 12_{c} est définie par un effort d'arrachement pour arracher la feuille 7 des protubérances 12ₐ, 12_{b}, 12_{c}. La fixation de la feuille 7 sur les protubérances 12ₐ bordant la gorge 10 permet un maintien du tube d'insertion 4 à l'intérieur de la gorge 10, et la fixation de la feuille 7 sur les protubérances 12_{b} bordant la gouttière 11 permet un maintien du câble d'alimentation 5 à l'intérieur de la gorge 11. Typiquement, l'effort d'arrachement entre la feuille 7 et les protubérances 12ₐ, 12_{b}, 12_{c} va de 0,001 N/mm² à 0,1 N/mm², de préférence de 0,005 N/mm² à 0,02 N/mm².

Dans le cadre de l'invention, l'effort d'arrachement entre la feuille 7 et les protubérances 12ₐ, 12_{b}, 12_{c} est inférieur à l'effort d'arrachement entre la feuille 7 et la plage périphérique 6ₚ du plateau 6. Ainsi, l'ouverture de l'emballage 1 par le praticien est aisée, tout en permettant de garantir la stérilité de l'endoscope médical 2 qui se trouve à l'intérieur de l'emballage 1 et son stockage de manière sûre. Plus précisément, l'effort d'arrachement entre la feuille 7 et les protubérances 12ₐ, 12_{b}, 12_{c} est inférieur d'au moins 30% par rapport à l'effort d'arrachement entre la feuille et la plage périphérique du plateau, c'est-à-dire que le ratio entre l'effort d'arrachement entre la feuille 7 et les protubérances 12ₐ, 12_{b}, 12_{c} et l'effort d'arrachement entre la feuille 7 et la plage périphérique 6ₚ du plateau 6 à isosurface (c'est-à-dire pour une même unité de surface) est inférieur ou égal à 0,7. De plus, le nombre, la forme, les dimensions, et la valeur de l'effort d'arrachement des protubérances 12ₐ, 12_{b}, 12_{c} peuvent être choisis par l'homme du métier en fonction des forces d'arrachement totales désirées et de manière à permettre un maintien du tube d'insertion 4 à l'intérieur de la gorge 10 et un maintien du câble d'alimentation 5 dans la gouttière 11.

La feuille 7 est réalisée en tout matériau permettant sa fixation au plateau 6 et permettant de garantir la stérilité de l'endoscope médical 2 en constituant une barrière stérile. En effet, la feuille 7 doit être perméable aux rayonnements béta et gamma pour permettre la stérilisation de l'emballage 1 contenant l'endoscope médical 2. De plus, la feuille 7 doit être imperméable aux microbes pour permettre de conserver l'endoscope médical 2 de manière stérile dans son emballage. Par exemple, la feuille 7 peut être réalisée en plastique ou en une combinaison de papier et de plastique. Selon un mode de réalisation préféré, le film est réalisé en matériau plastique, notamment thermoplastique, tel que du polyamide ou du polyéthylène comme le Tyvek^{®} commercialisé par la société Dupont de Nemours par exemple.

Selon un mode de réalisation particulier de l'invention représenté à la figure 5, l'emballage 1 comprend en outre deux lignes d'arrêt 13 aménagées dans la plage périphérique 6ₚ. Ces lignes d'arrêt 13 sont situées de part et d'autres de la gorge 10, et de préférence appartiennent à un même axe perpendiculaire à l'axe longitudinal L de la gorge 10. Ainsi, ces lignes d'arrêt traversent les deux côtés de la plage périphérique 6ₚ situés de part et d'autre de la gorge 10. Ainsi, lorsque le praticien ouvre l'emballage 1, cette ouverture (consistant à détacher la feuille 7 du plateau 6) peut se faire jusqu'à ces deux lignes d'arrêt 13. L'ouverture doit alors être suffisamment grande pour permettre de sortir aisément l'endoscope médical 2 de son emballage 1. Avantageusement, l'endoscope médical peut être replacé dans l'emballage 1 facilement par le praticien, si besoin.

Selon un mode de réalisation particulier, l'emballage 1 comprend en outre une surface adhésive 14 sur la face interne 6ᵢ du plateau 6, de préférence en vis-à-vis de la plage périphérique 6ₚ tel qu'illustré à la figure 9, et/ou recouvrant en partie de la plage périphérique 6ₚ. La surface adhésive 14 peut être continue ou discontinue. De préférence, la surface adhésive 14 se trouve sous la forme d'un contour allant d'une ligne d'arrêt 13 à l'autre, dans la partie de la face interne 6ᵢ du plateau 6 destinée à être ouverte, c'est-à-dire du côté de la cavité 9. Alternativement, comme illustré à la figure 9, cette surface adhésive 14 se trouve sur tout le pourtour de l'emballage, c'est-à-dire sur tout le long de la plage périphérique 6ₚ. La surface adhésive 14 est soit contigüe de la plage périphérique 6ₚ, soit à proximité de la plage périphérique 6ₚ, et, quel que soit le mode de réalisation, éventuellement recouvrant partiellement la plage périphérique 6ₚ. La surface adhésive 14 est de préférence positionnée en bordure de la cavité 9. Avantageusement, la surface adhésive 14 est positionnée entre la plage périphérique 6ₚ et le logement 8. Selon ce mode de réalisation, la surface adhésive 14 est recouverte d'un film pelable. Ainsi selon ce mode de réalisation, le praticien peut ouvrir l'emballage 1 en totalité ou jusqu'aux deux lignes d'arrêt 13, utiliser l'endoscope médical 2 selon son besoin, le replacer dans l'emballage, ôter le film pelable et refermer l'emballage 1 en mettant en contact la surface adhésive 14 avec la feuille 7. Selon les besoins du praticien, l'emballage peut être ouvert ou refermé.

Selon un mode de réalisation particulier, l'emballage 1 selon l'invention comprend une fente d'accrochage 15 dans la plage périphérique 6ₚ, afin de pouvoir suspendre l'emballage 1. De préférence, cette fente 15 est oblongue. De préférence, cette fente 15 est située dans la partie de la plage périphérique 6ₚ située en vis-à-vis de la cavité 9 destinée à recevoir la poignée de commande 3.

L'invention concerne également un procédé d'emballage d'un endoscope médical 2 comprenant les étapes suivantes :
a) disposer d'un endoscope médical 2 comportant une poignée de commande 3, un tube d'insertion 4, et éventuellement un câble d'alimentation 5,
b) disposer d'un plateau 6 pourvu d'une face interne 6ᵢ présentant une plage périphérique 6ₚ, ledit plateau 6 comportant sur sa face interne 6ᵢ un logement 8 destiné à recevoir un endoscope médical 2, le logement 8 étant composé d'une cavité 9 destinée à recevoir la poignée de commande 3, d'une gorge 10 rectiligne destinée à recevoir le tube d'insertion 4, et éventuellement d'une gouttière 11 destinée à recevoir le câble d'alimentation 5, la gorge 10 présentant une section supérieure à la section du tube d'insertion 4 pour permettre sa libre insertion, ledit plateau 6 comprenant des protubérances 12ₐ s'étendant en saillie à partir de sa face interne 6ᵢ et disposées de part et d'autre de la gorge 10,
c) disposer d'une feuille 7 étanche et thermoscellable,
d) placer l'endoscope médical 2 dans le logement 8,
e) mettre en contact la feuille 7 thermoscellable avec la plage périphérique 6ₚ et avec les protubérances 12ₐ, chauffer à une température de scellage, appliquer une pression de scellage périphérique sur la plage périphérique 6ₚ permettant un scellage hermétique de la feuille 7 sur la plage périphérique 6ₚ pour obtenir un effort d'arrachement, et appliquer une pression de scellage central sur les protubérances 12ₐ permettant une fixation de la feuille 7 sur les protubérances 12ₐ pour obtenir un effort d'arrachement, la pression de scellage périphérique et la pression de scellage central étant telles que l'effort d'arrachement entre la feuille 7 et les protubérances 12ₐ est inférieur d'au moins 30% par rapport à l'effort d'arrachement entre la feuille 7 et la plage périphérique 6ₚ.

A l'étape b), le plateau 6 est tel que décrit ci-dessus pour l'emballage 1 selon l'invention.

A l'étape c), la feuille 7 est telle que décrite ci-dessus pour l'emballage 1 selon l'invention.

A l'étape e), le chauffage peut être réalisé par conduction, rayonnement IR, faisceau laser, ou ultrason à l'aide d'une sonotrode par exemple. La température de scellage est définie comme la température permettant le thermoscellage entre le plateau 6 et la feuille 7. Cette température dépend des matériaux employés pour la réalisation du plateau 6 et de la feuille 7, et peut être déterminée par l'homme du métier.

A l'étape e), la pression est appliquée grâce à une sonotrode par exemple.

A l'étape e), une pression de scellage périphérique est appliquée sur la feuille 7 et la plage périphérique 6ₚ du plateau 6, de préférence de manière concomitante au chauffage, ce qui permet un scellage hermétique sur tout le pourtour de l'emballage 1, entre la feuille 7 et la plage périphérique 6ₚ du plateau 6. Ce chauffage combiné à l'application de la pression périphérique permet le thermoscellage entre la plage périphérique 6ₚ du plateau 6 et la feuille 7.

Comme décrit ci-dessus, le plateau 6 peut comporter au moins une protubérance 12_{b} bordant la gouttière 11 et/ou au moins une protubérance 12_{c} aménagée dans la gouttière 11. Selon ce mode de réalisation, la pression de scellage central est également appliquée sur les protubérances 12_{b} bordant la gouttière 11 et/ou les protubérances 12c aménagées dans la gouttière 11, pour permettre la fixation de la feuille 7 sur toutes les protubérances 12ₐ, 12_{b}, 12_{c} et obtenir un effort d'arrachement. Selon ce mode de réalisation, la pression de scellage périphérique et la pression de scellage central sont telles que l'effort d'arrachement entre la feuille 7 et les protubérances 12ₐ, 12_{b}, 12_{c} est inférieur d'au moins 30% par rapport à l'effort d'arrachement entre la feuille 7 et la plage périphérique 6ₚ.

A l'étape e), une pression de scellage central est appliquée sur la feuille 7 et les protubérances 12ₐ, 12_{b}, 12_{c} s'étendant en saillie du plateau 6, de préférence de manière concomitante au chauffage, ce qui permet de fixer la feuille 7 sur la face supérieure 12ₛ des protubérances 12. Ainsi, la feuille 7 est fixée aux protubérances 12ₐ, 12_{b}, 12_{c}, et plus précisément à la face supérieure 12ₛ des protubérances 12ₐ, 12_{b}, 12_{c}.

Dans le cadre de l'invention, l'effort d'arrachement entre la feuille 7 et la plage périphérique 6ₚ du plateau 6 est supérieur à l'effort d'arrachement entre la feuille 7 et les protubérances 12ₐ, 12_{b}, 12_{c}, d'au moins 30%. Pour ce faire, la pression de scellage central est inférieure à la pression de scellage périphérique. Selon un premier mode de réalisation, à l'étape b), la plage périphérique 6ₚ et les protubérances 12ₐ, 12_{b}, 12_{c} s'étendent en saillie selon une même hauteur par rapport à la surface interne 6ᵢ du plateau 6. Selon ce mode de réalisation, la pression appliquée à l'étape e) au niveau de la plage périphérique 6ₚ est alors plus élevée que la pression appliquée au niveau des protubérances 12ₐ, 12_{b}, 12_{c}. Selon un second mode de réalisation, à l'étape b), les protubérances 12ₐ, 12_{b}, 12_{c} sont en saillie selon une hauteur inférieure à celle de la plage périphérique 6ₚ par rapport à la surface interne 6ᵢ du plateau 6. Selon ce mode de réalisation, à l'étape e), la pression appliquée au niveau de la plage périphérique 6ₚ peut être identique à celle appliquée au niveau des protubérances 12ₐ, 12_{b}, 12_{c}.

## Revendications

1. Emballage (1) d'un endoscope médical (2) comportant une poignée de commande (3) et un tube d'insertion (4) , ledit emballage (1) comprenant :
- un plateau (6) pourvu d'une face interne (6ᵢ) présentant une plage périphérique (6ₚ), ledit plateau (6) comportant sur sa face interne (6ᵢ) un logement (8) recevant l'endoscope médical (2), le logement (8) comprenant une cavité (9) recevant la poignée de commande (3), et une gorge (10) rectiligne recevant le tube d'insertion (4), la gorge (10) présentant une section supérieure à la section du tube d'insertion (4) pour permettre sa libre insertion,
- une feuille (7) perméable aux gaz et aux rayonnements béta et gamma, imperméable aux microbes, scellée continument et hermétiquement à la plage périphérique (6ₚ) de la face interne (6ᵢ) du plateau (6) selon un effort d'arrachement,
**caractérisé en ce que** le plateau (6) comprend des protubérances (12ₐ) s'étendant en saillie à partir de sa face interne (6ᵢ) et disposées de part et d'autre de la gorge (10),
**en ce que** la feuille (7) est fixée aux protubérances (12ₐ) selon un effort d'arrachement,
et **en ce que** le ratio entre l'effort d'arrachement entre la feuille (7) et les protubérances et l'effort d'arrachement entre la feuille (7) et la plage périphérique (6ₚ) à isosurface est inférieur ou égale à 0,7.

2. Emballage (1) selon la revendication 1, dans lequel les protubérances (12ₐ) sont positionnées en bordure de la gorge (10).

3. Emballage (1) selon l'une quelconque des revendications précédentes, dans lequel les protubérances (12ₐ) sont réparties le long de la gorge (10).

4. Emballage (1) selon l'une quelconque des revendications précédentes, dans lequel les protubérances (12ₐ) sont disposées alternativement d'un côté ou de l'autre de la gorge (10).

5. Emballage (1) selon l'une quelconque des revendications précédentes, dans lequel le logement (8) comprend une gouttière (11) recevant un câble d'alimentation (5) de l'endoscope médical (2).

6. Emballage (1) selon la revendication précédente, selon lequel au moins une protubérance (12_{b}) est positionnée en bordure de la gouttière (11).

7. Emballage selon la revendication 5 ou 6, selon lequel au moins une protubérance (12_{c}) est présente dans la gouttière (11) afin d'enrouler le câble d'alimentation (5).

8. Emballage (1) selon l'une quelconque des revendications précédentes, dans lequel les protubérances (12ₐ, 12_{b}, 12_{c}) sont des plots ou des nervures.

9. Emballage (1) selon l'une quelconque des revendications précédentes, comprenant deux lignes d'arrêt aménagées dans les parties de la plage périphérique (6ₚ) situées de part et d'autre de la gorge (10).

10. Emballage (1) selon l'une quelconque des revendications précédentes, dans lequel le logement (8) comporte des clips pour maintenir en position la poignée de commande (3).

11. Emballage (1) selon l'une quelconque des revendications précédentes, selon lequel le logement (8) comprend une alvéole (11ₐ) recevant un connecteur (5_{c}) du câble d'alimentation (5) de l'endoscope médical (2).

12. Emballage (1) selon l'une quelconque des revendications précédentes, comprenant une fente d'accrochage (15) dans la plage périphérique (6ₚ).

13. Emballage selon l'une quelconque des revendications précédentes comprenant en outre une surface adhésive (14) sur la face interne (6ᵢ) du plateau (6), recouverte d'un film pelable.

14. Procédé d'emballage d'un endoscope médical (2) comprenant les étapes suivantes :
a) disposer d'un endoscope médical (2) comportant une poignée de commande (3), un tube d'insertion (4), et éventuellement un câble d'alimentation (5),
b) disposer d'un plateau (6) pourvu d'une face interne (6ᵢ) présentant une plage périphérique (6ₚ), ledit plateau (6) comportant sur sa face interne (6ₚ) un logement (8) destiné à recevoir un endoscope médical (2), le logement (8) étant composé d'une cavité (9) destinée à recevoir la poignée de commande (3), d'une gorge (10) rectiligne destinée à recevoir le tube d'insertion (4), et éventuellement d'une gouttière (11) destinée à recevoir le câble d'alimentation (5), la gorge (10) présentant une section supérieure à la section du tube d'insertion (4) pour permettre sa libre insertion, ledit plateau (6) comprenant des protubérances (12ₐ) s'étendant en saillie à partir de sa face interne (6ᵢ) et disposées de part et d'autre de la gorge (10),
c) disposer d'une feuille (7) perméable aux gaz et aux rayonnements béta et gamma, imperméable aux microbes et thermoscellable,
d) placer l'endoscope médical (2) dans le logement (8),
e) mettre en contact la feuille (7) thermoscellable avec la plage périphérique (6ₚ) et avec les protubérances (12ₐ), chauffer à une température de scellage, appliquer une pression de scellage périphérique sur la plage périphérique (6ₚ) permettant un scellage hermétique de la feuille (7) sur la plage périphérique (6ₚ) pour obtenir un effort d'arrachement, et appliquer une pression de scellage central sur les protubérances (12ₐ) permettant une fixation de la feuille (7) sur les protubérances (12ₐ) pour obtenir un effort d'arrachement, la pression de scellage périphérique et la pression de scellage central étant telles que l'effort d'arrachement entre la feuille (7) et les protubérances (12ₐ) est inférieur d'au moins 30% par rapport à l'effort d'arrachement entre la feuille (7) et la plage périphérique (6ₚ).

15. Procédé selon la revendication précédente, selon lequel, à l'étape b), la plage périphérique (6ₚ) et les protubérances (12ₐ) s'étendent en saillie selon une même hauteur par rapport à la surface interne (6ᵢ) du plateau.

16. Procédé selon la revendication 14, selon lequel, à l'étape b), les protubérances (12ₐ) sont en saillie selon une hauteur inférieure à celle de la plage périphérique (6ₚ) par rapport à la surface interne (6ᵢ) du plateau.

## Patentansprüche

1. Verpackung (1) eines medizinischen Endoskops (2), das einen Steuergriff (3) und einen Einführschlauch (4) umfasst, die Verpackung (1) umfassend:
- Platte (6), die mit einer Innenseite (6ᵢ) versehen ist, die einen umlaufenden Randbereich (6ₚ) aufweist, wobei die Platte (6) auf ihrer Innenseite (6ᵢ) eine Aufnahme (8) aufweist, die das medizinische Endoskop (2) aufnimmt, wobei die Aufnahme (8) eine Ausnehmung (9), die den Steuergriff (3) aufnimmt, und eine geradlinige Rille (10), die den Einführschlauch (4) aufnimmt, umfasst, wobei die Rille (10) einen Querschnitt aufweist, der größer als der Querschnitt des Einführschlauchs (4) ist, um dessen freies Einführen zu ermöglichen,
- Folie (7), durchlässig für Gase und Beta- und Gammastrahlung, undurchlässig für Mikroben, durchgehend und hermetisch mit dem umlaufenden Randbereich (6ₚ) der Innenseite (6ᵢ) der Platte (6) mit einer Abzugskraft gesiegelt,
**dadurch gekennzeichnet, dass** die Platte (6) Vorsprünge (12ₐ) aufweist, die von ihrer Innenseite (6ᵢ) vorstehen und auf beiden Seiten der Rille (10) angeordnet sind,
**dadurch, dass** die Folie (7) an den Vorsprüngen (12ₐ) mit einer Abzugskraft befestigt ist,
**und dadurch, dass** das Verhältnis zwischen der Abzugskraft zwischen der Folie (7) und den Vorsprüngen und der Abzugskraft zwischen der Folie (7) und dem umlaufenden Randbereich (6ₚ) bei gleicher Fläche kleiner oder gleich 0,7 ist.

2. Verpackung (1) nach Anspruch 1, wobei die Vorsprünge (12ₐ) am Rand der Rille (10) angeordnet sind.

3. Verpackung (1) nach einem der vorstehenden Ansprüche, wobei die Vorsprünge (12ₐ) entlang der Rille (10) verteilt sind.

4. Verpackung (1) nach einem der vorstehenden Ansprüche, wobei die Vorsprünge (12ₐ) abwechselnd auf der einen oder anderen Seite der Rille (10) angeordnet sind.

5. Verpackung (1) nach einem der vorstehenden Ansprüche, wobei die Aufnahme (8) eine Rinne (11) aufweist, die ein Versorgungskabel (5) des medizinischen Endoskops (2) aufnimmt.

6. Verpackung (1) nach dem vorstehenden Anspruch, wobei mindestens ein Vorsprung (12_{b}) am Rand der Rinne (11) angeordnet ist.

7. Verpackung nach Anspruch 5 oder 6, wobei mindestens ein Vorsprung (12_{c}) in der Rinne (11) vorhanden ist, um das Versorgungskabel (5) aufzuwickeln.

8. Verpackung (1) nach einem der vorstehenden Ansprüche, wobei die Vorsprünge (12ₐ, 12_{b}, 12_{c}) Noppen oder Rippen sind.

9. Verpackung (1) nach einem der vorstehenden Ansprüche, umfassend zwei Haltelinien, die in den auf beiden Seiten der Rille (10) befindlichen Teilen des umlaufenden Randbereichs (6ₚ) ausgebildet sind.

10. Verpackung (1) nach einem der vorstehenden Ansprüche, wobei die Aufnahme (8) Clips aufweist, um den Steuergriff (3) in Position zu halten.

11. Verpackung (1) nach einem der vorstehenden Ansprüche, wobei die Aufnahme (8) eine Aussparung (11ₐ) umfasst, die einen Verbinder (5_{c}) des Versorgungskabels (5) des medizinischen Endoskops (2) aufnimmt.

12. Verpackung (1) nach einem der vorstehenden Ansprüche, umfassend einen Aufhängeschlitz (15) im umlaufenden Randbereich (6ₚ).

13. Verpackung nach einem der vorstehenden Ansprüche, ferner umfassend eine Klebefläche (14) auf der Innenseite (6ᵢ) der Platte (6), die mit einer abziehbaren Folie abgedeckt ist.

14. Verfahren zum Verpacken eines medizinischen Endoskops (2), umfassend die folgenden Schritte:
a) Bereitstellen eines medizinischen Endoskops (2), umfassend einen Steuergriff (3), einen Einführschlauch (4) und gegebenenfalls ein Versorgungskabel (5),
b) Bereitstellen einer Platte (6), die mit einer Innenseite (6ᵢ) versehen ist mit einem umlaufenden Randbereich (6ₚ), wobei die Platte (6) auf ihrer Innenseite (6ₚ) eine Aufnahme (8) umfasst, die zur Aufnahme eines medizinischen Endoskops (2) bestimmt ist, wobei die Aufnahme (8) aus einer Ausnehmung (9), die zur Aufnahme des Steuergriffs (3) bestimmt ist, einer geradlinigen Rille (10), die zur Aufnahme des Einführschlauchs (4) bestimmt ist, und gegebenenfalls einer Rinne (11), die zur Aufnahme des Versorgungskabels (5) bestimmt ist, besteht, wobei die Rille (10) einen Querschnitt aufweist, der größer als der Querschnitt des Einführschlauchs (4) ist, um dessen freies Einführen zu ermöglichen, wobei die Platte (6) Vorsprünge (12ₐ) umfasst, die von ihrer Innenseite (6ᵢ) hervorstehen und auf beiden Seiten der Rille (10) angeordnet sind,
c) Bereitstellen einer Folie (7), die für Gase und Beta- und Gammastrahlung durchlässig, für Mikroben undurchlässig und heißsiegelbar ist,
d) Anordnen des medizinischen Endoskops (2) in der Aufnahme (8),
e) Inkontaktbringen der heißsiegelbaren Folie (7) mit dem umlaufenden Randbereich (6ₚ) und mit den Vorsprüngen (12ₐ), Erhitzen auf eine Siegeltemperatur, Ausüben eines umlaufenden Siegeldrucks auf den umlaufenden Randbereich (6ₚ), das eine hermetische Versiegelung der Folie (7) auf dem umlaufenden Randbereich (6ₚ) ermöglicht, um eine Abzugskraft zu erhalten, und Ausüben eines zentralen Siegeldrucks auf die Vorsprünge (12ₐ), die eine Befestigung der Folie (7) an den Vorsprüngen (12ₐ) ermöglicht, um eine Abzugskraft zu erhalten, wobei der umlaufende Siegeldruck und der zentrale Siegeldruck derart sind, dass die Abzugskraft zwischen der Folie (7) und den Vorsprüngen (12ₐ) um mindestens 30 % geringer ist als die Abzugskraft zwischen der Folie (7) und dem umlaufenden Randbereich (6ₚ).

15. Verfahren nach dem vorstehenden Anspruch, wobei in Schritt b) der umlaufende Randbereich (6ₚ) und die Vorsprünge (12ₐ) um eine gleiche Höhe in Bezug auf die Innenseite (6ᵢ) der Platte vorstehen.

16. Verfahren nach Anspruch 14, wobei in Schritt b) die Vorsprünge (12ₐ) in Bezug auf die Innenseite (6ᵢ) der Platte um eine Höhe vorstehen, die geringer ist als diejenige des umlaufenden Randbereichs (6ₚ).

## Claims

1. A packaging (1) for a medical endoscope (2) including an operating handle (3) and an insertion tube (4), said packaging (1) comprising:
- a tray (6) with an inner face (6ᵢ) having a peripheral region (6ₚ), said tray (6) including, on its inner face (6ᵢ), a housing (8) that receives the medical endoscope (2), the housing (8) comprising a cavity (9) that receives the operating handle (3), and a straight groove (10) that receives the insertion tube (4), the groove (10) having a cross-section greater than that of the insertion tube (4) to allow for its free insertion,
- a sheet (7) that is permeable to gases and to beta and gamma radiation, impermeable to microbes, and continuously and hermetically sealed to the peripheral region (6ₚ) of the inner face (6ᵢ) of the tray (6) with a specified peel load,
**characterized in that** the tray (6) comprises protrusions (12ₐ) extending outward from its inner face (6ᵢ) and arranged on either side of the groove (10),
**in that** the sheet (7) is secured to the protrusions (12ₐ) with a specified peel load,
**and in that** the ratio of the peel load between the sheet (7) and the protrusions to the peel load between the sheet (7) and the isosurface peripheral region (6ₚ) is less than or equal to 0.7.

2. The packaging (1) according to claim 1, wherein the protrusions (12ₐ) are positioned at the edge of the groove (10).

3. The packaging (1) according to any of the preceding claims, wherein the protrusions (12ₐ) are distributed along the groove (10).

4. The packaging (1) according to any of the preceding claims, wherein the protrusions (12ₐ) are arranged alternately on one side or the other of the groove (10).

5. The packaging (1) according to any of the preceding claims, wherein the housing (8) comprises a channel (11) that receives a power cable (5) of the medical endoscope (2).

6. The packaging (1) according to the preceding claim, wherein at least one protrusion (12_{b}) is positioned at the edge of the channel (11).

7. The packaging according to claim 5 or 6, wherein at least one protrusion (12_{c}) is present in the channel (11) in order to wind the power cable (5).

8. The packaging (1) according to any of the preceding claims, wherein the protrusions (12ₐ, 12_{b}, 12_{c}) are studs or ribs.

9. The packaging (1) according to any of the preceding claims, comprising two stop lines formed in the portions of the peripheral region (6ₚ) located on either side of the groove (10).

10. The packaging (1) according to any of the preceding claims, wherein the housing (8) includes clips for holding the operating handle (3) in place.

11. The packaging (1) according to any of the preceding claims, wherein the housing (8) comprises a recess (11ₐ) that receives a connector (5_{c}) of the power cable (5) of the medical endoscope (2).

12. The packaging (1) according to any of the preceding claims, comprising a hooking slot (15) in the peripheral region (6ₚ).

13. The packaging according to any of the preceding claims, further comprising an adhesive surface (14) on the inner face (6ᵢ) of the tray (6), covered with a peelable film.

14. A method for packaging a medical endoscope (2) comprising the following steps:
a) having a medical endoscope (2) including an operating handle (3), an insertion tube (4), and, optionally, a power cable (5),
b) having a tray (6) with an inner face (6ᵢ) having a peripheral region (6ₚ), said tray (6) including, on its inner face (6ₚ), a housing (8) designed to receive a medical endoscope (2), the housing (8) consisting of a cavity (9) designed to receive the operating handle (3), a straight groove (10) designed to receive the insertion tube (4), and optionally a channel (11) designed to receive the power cable (5), the groove (10) having a cross-section greater than that of the insertion tube (4) to allow for its free insertion, said tray (6) comprising protrusions (12ₐ) extending outward from its inner face (6ᵢ) and arranged on either side of the groove (10),
c) having a sheet (7) that is permeable to gases and to beta and gamma radiation, impermeable to microbes, and heat-sealable,
d) placing the medical endoscope (2) in the housing (8),
e) bringing the heat-sealable sheet (7) into contact with the peripheral region (6ₚ) and with the protrusions (12ₐ), heating to a sealing temperature, applying a peripheral sealing pressure to the peripheral region (6ₚ) to hermetically seal the sheet (7) to the peripheral region (6ₚ) to obtain a peel load, and applying a central sealing pressure to the protrusions (12ₐ) to secure the sheet (7) to the protrusions (12ₐ) to obtain a peel load, the peripheral sealing pressure and the central sealing pressure being such that the peel load between the sheet (7) and the protrusions (12ₐ) is at least 30% lower than the peel load between the sheet (7) and the peripheral region (6ₚ).

15. The method according to the preceding claim, wherein, in step b), the peripheral region (6ₚ) and the protrusions (12ₐ) extend outward by the same height relative to the inner surface (6ᵢ) of the tray.

16. The method according to claim 14, wherein, in step b), the protrusions (12ₐ) project to a height less than that of the peripheral region (6ₚ) relative to the inner surface (6ᵢ) of the tray.
